Europäisches Patentamt

**European Patent Office**

**Office européen des brevets**

(11) Publication number: **0 255 556 B1**

(12) **EUROPEAN PATENT SPECIFICATION**

(45) Date of publication of patent specification: **26.02.92** (51) Int. Cl.⁵: **A61F 2/66**

(21) Application number: **86110954.4**

(22) Date of filing: **08.08.86**

(54) **Prosthetic joint.**

(43) Date of publication of application:
**10.02.88 Bulletin 88/06**

(45) Publication of the grant of the patent:
**26.02.92 Bulletin 92/09**

(84) Designated Contracting States:
**BE CH DE FR GB IT LI NL SE**

(56) References cited:
DE-C- 384 684          DE-C- 813 191
DE-U- 1 891 505        GB-A- 2 124 085
US-A- 2 439 195        US-A- 2 617 115

(73) Proprietor: **Fleischauer, K.E.**
**2031 S.E. 33rd Street**
**Okeechobee FL 33472(US)**

(72) Inventor: **Fleischauer, K.E.**
**2031 S.E. 33rd Street**
**Okeechobee FL 33472(US)**

(74) Representative: **Patentanwälte RUFF, BEIER**
**und SCHÖNDORF**
**Neckarstrasse 50**
**W-7000 Stuttgart 1(DE)**

Rank Xerox (UK) Business Services

## Description

### Background of the Invention

#### 1. Field of the Invention

This invention relates generally to prosthetic joints, and specifically to a prosthetic ankle which is inexpensive, durable, life-like in operation, and can be easily and inexpensively installed into rigid prosthetic leg devices.

#### 2. Description of the Prior Art

The ankle portion of prosthetic devices for both below-knee and above-knee amputees is conventionally rigid. A piece of shock absorbing substance such as rubber may be provided at the heel of the artificial foot to both absorb shock during walking and to provide some flexibility. However, this construction is a poor substitute for the natural movement of an ankle. The natural rocking motion afforded by an ankle smooths the walking motion and the body parts associated therewith help to absorb the shock forces that are associated with walking.

Prosthetic ankles have been provided for the amputee which seek to simulate the movement of the human ankle. These are quite expensive and usually require the purchase of a new leg and foot as they are commonly designed to cooperate with the ankle. These devices typically provide only a limited range of mobility, on the order of $3\frac{1}{2}°$ forward of the vertical axis and $1°$ backward. Previous ankle designs also provided little in the way of sideways give and therefore made walking laterally or sideways to an inclined surface difficult. Moreover, such designs often have no means for returning the foot to its natural position, perpendicular to the leg, without the application of external force. Those devices which do have internal biasing means are often either too restrictive and do not provide sufficient play or give, or are too loose and tend to flap about. Moreover, the biasing resistance which they provide is relatively constant over the full range of movement, limited though it is. It would be desirable to have more give at angles close to the vertical axis where fluctuations are common and more biasing at larger angles where greater support is needed. Previous prosthetic ankles have been very susceptible to wear and tend to work loose or deteriorate markedly with use. These failures of the prior art all contribute to a marked safety hazard in the use of prosthetic devices. Wobbling, and even falling, are everyday fears for the wearers of prosthetic leg devices.

The prior art devices teaches a prosthetic ankle devices for an artificial leg (US-A-2 617 115), having a leg portion and a foot portion, are pivotally connected for limited relative pivotal movements by means of an ankle joint structure. It comprises basically a pair of plates being separately fixed by bolts to the foot and leg portion and hingedly connected to each other. The hinge is active in only one direction, namely forward and backward in walking direction. The full shearing strength to the pivot pin will lead to wear of the pin and may loosen the hinge and cause a break of the pin. Additionally to this hinged ankle joint structure, there is by every gait a typical clatter of the ankle joint which causes additional wear of the hinge.

By the document DE-U-1 891 505 another ankle joint structure for an artificial leg is known, which comprises an elastomeric plate between the leg part and the foot part held together with a connecting bolt mounted to the elastomeric plate perpendicular which is fitted in the heel of the foot part through an elastomeric element and a nut fixed in the artificial leg part.

The document US-A 2 439 195 teaches also a various kind of ankle joint structure which is also held together by a perpendicular mounted bolt.

The connecting bolt of the two above mentioned ankle joints have the only function of a hinge. Because there is no preferred hinge direction (forward - backward) this joint is uncomfortable and unsafe for the user. Moreover it is the only connection between the foot and the leg part. If the connecting bolt fails the wearer will have a dangerous prosthetic device fail.

The present invention provides a prosthetic ankle joint which is easily installed in conventional rigid prosthetic legs. The joint is inexpensively made and installed, and provides an extended range of movement, similar to that of the human ankle. The joint is internally biased to return the foot to a position perpendicular to the leg, and the biasing increases as bending increases. It is made of durable materials which can withstand the substantial forces which are placed on such a device.

### Summary of the Invention

It is an object of the present invention to provide a prosthetic ankle device which is easily incorporated into conventional, rigid artificial legs.

It is another object of the present invention to provide a prosthetic ankle device which is inexpensively made and installed in conventional, rigid artificial legs.

It is yet another object of the present invention to provide a prosthetic ankle device which closely simulates the range of motion afforded by the human ankle.

It is still another object of the present invention to provide a prosthetic ankle device which is biased to return the foot to a position perpendicular to the leg portion.

It is another object of the present invention to provide a prosthetic ankle device which provides relatively small resistance at leg-to-foot angles close to the perpendicular but affords greater biasing resistance as bending increases.

It is still another object of the present invention to provide a prosthetic ankle device which is durable when exposed to the severe forces which act on such a device.

It is yet another object of the present invention to provide a prosthetic ankle device which will safely secure the foot portion to the leg portion.

These and other objects are accomplished by providing a prosthetic ankle device for movably attaching a lower leg part to a foot part, the device to displace at least a part of a prosthetic leg, the device separating the lower leg part and the foot part from one another, the lower leg part and foot part defining facing surfaces, the device comprising:

upper and lower plates conforming in shape and adapted for attachment to said facing surfaces of said lower leg and foot parts, respectively,

said upper and lower plates together forming hinge means connecting said plates, said hinge means defining a lateral axis of rotation for the foot portion and the lower leg portion relative to one another, said hinge means being divided to form a recess in said upper and lower plates,

resilient biasing means disposed between said plates on both sides of said lateral axis for progressively resisting rotation about said axis, whereby natural foot rotation about an ankle is simulated with respect to cushioning of foot strikes without risking loss of balance; and

auxiliary bolt means for securing said foot part adjustably to said lower leg part extending longitudinally through bores in the foot and leg parts and through said recess of the hinged plates, which bores are aligned with said recess, and extending substantially through the lateral axis, while allowing said rotation.

Accordingly the ankle device is formed by opposing upper and lower plates adapted for attachment to the lower leg portion and foot portions, respectively, of a conventional rigid prosthetic leg at its ankle portion. The plates are attached to each other by interfitted hinge means such that the natural rotations of the foot relative to the leg are permitted. The hinge means is preferably formed of projecting semicylindrical rings and grooves adapted to receive the rings. The rings have hollow cores which align when the rings are interfitted so as to receive a pin or pins which forms a hinge axis and holds the joint securely together.

Biasing means are located on the fore and aft side of the hinge joint which progressively resist rotation about the hinge axis. Each biasing member preferably comprises a rubber cylinder, the axis of which is perpendicular to the hinge axis and the top and bottom faces of which rest in seats formed in the plates. The biasing members further comprise springs, the coils of which loosely wrap the rubber cylinder. Means for stopping rotation are also provided. These means preferably comprise a rubber grommet further encircling both the spring and the cylinder.

The biasing means acts to return the foot to a position perpendicular to the leg, counteracting rotational forces with progressive resistance. The biasing means provides minor resistance to rotation about the hinge axis when the rotation is minor, such that the leg is substantially perpendicular to the foot. When bending becomes more extreme, however, increased resistance is provided as the cylinder acts on the spring and finally as the grommet is engaged by the plates. The hinge members and pins are precision formed with sufficient space therebetween to allow a small lateral rotation of the foot.

The ankle is provided with auxiliary bolt means, which extends through the recess, preferably designed as slots in the plates from a bore in the foot, into a bore in the leg. The bolt head is seated on a grommet, and the bolt passes through a slot in the plates which extends fore and aft along a line perpendicular to the hinge axis and passing through the midpoint thereof. The grommet, slots and bores provide sufficient give around the bolt so that the afore described motions are permitted. The auxiliary bolt means provides an added safety measure such that should a pin shear, the foot will remain secured to the leg by the auxiliary bolt means.

Brief Description of the Drawings

There are shown in the drawings embodiments which are presently preferred. It should be understood, however, that the invention is not limited to the precise arrangements and instrumentalities shown.

Fig. 1 is a side elevation of a prosthetic ankle device according to this invention.

Fig. 2 is an exploded perspective view of the prosthetic ankle device shown in Fig. 1.

Fig. 3 is a cross-section of the prosthetic ankle device taken along the line 3-3 in Fig. 4.

Fig. 4 is a cross-section of the prosthetic ankle device taken along the line 4-4 in Fig. 1.

Fig. 5 is a cross-section of the prosthetic ankle device taken along the line 5-5 in Fig. 3.

Detailed Description of the Preferred Embodiments

This invention provides a prosthetic ankle device which is inexpensively and easily installed in conventional rigid prosthetic legs. It provides fore and aft biasing such that the foot returns to a position perpendicular to the leg when no force is acting on it. Greater biasing forces are provided when the angular relationship between the foot and the leg is substantially off the perpendicular. A small lateral rotation of the foot is provided as by the human ankle. The construction materials are durable and can readily withstand the severe forces acting on the ankle. A safety feature can be provided so that the foot would be securely held to the leg even should an ankle element break.

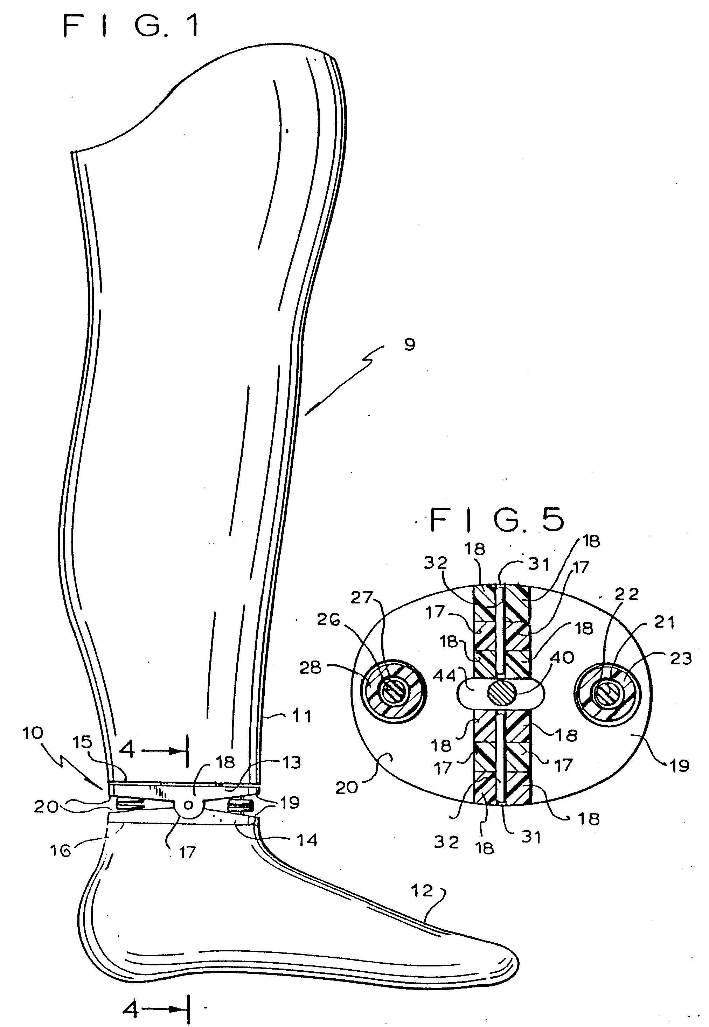

The manner in which the preceding features and advantages are provided will be described with reference to the drawings. Like numbers in the drawings refer to like elements in the various figures. With reference to Fig. 1, a prosthetic leg 9 has a leg portion 11, an ankle 10, and a foot portion 12. The leg 11 and foot 12 are such as would be found in a conventional rigid prosthetic leg, where they would otherwise form a single rigid member. The ankle 10 has an upper plate 13 secured to the lower leg surface 15 and a lower plate 14 secured to an upper foot surface 16 in a manner to be described below.

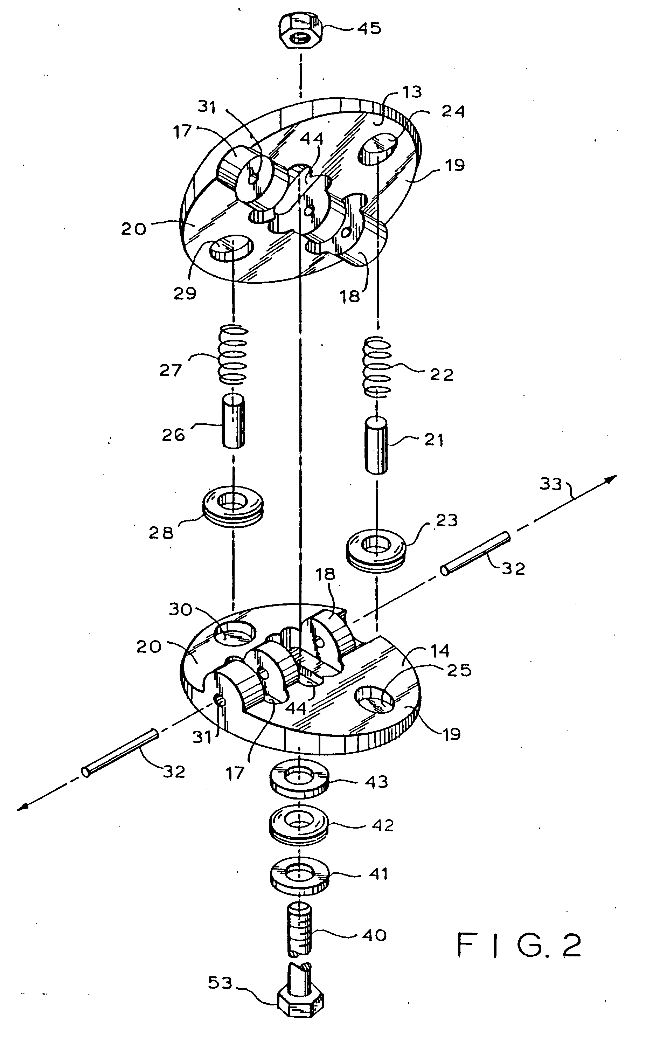

With reference now also to Figure 2, the plates have hinge means formed therein which preferably comprises projecting semicylindrical rings 18 with hollow cores 31, the rings 18 alternating with recessed grooves 17 adapted to receive the rings from the opposing plate. The rings of the upper plate are disposed so as to be received by the grooves of the lower plate and to interfit with the rings of the lower plate. Similarly, the rings of the lower plate are received by the grooves of the upper plate when the rings are interfitted. When the rings are so interfitted, the cores 31 of the rings 18 align so as to form a bore through which are positioned pins 32 to hingeably secure the joint together. The location of the hinge axis 33 so formed should substantially duplicate the location of the axis of the human ankles, and more or less divides the plates into a fore half 19 and an aft half 20.

The plates 13 and 14 are preferably sloped fore and aft from the hinge axis such that the plates are narrower at their front and rear extremities. The opposing surfaces of the fore halves 19 and the aft halves 20 define included angles which represent the maximum allowable rotation about the hinge axis.

Biasing means are located fore and aft of the hinge axis 33. The biasing means preferably comprises, as forward of the hinge axis 33, a rubber cylinder 21 that is disposed perpendicularly to the hinge axis and preferably located on a line that bisects the hinge axis. A spring 22 is disposed over the cylinder 21 such that the coils of the spring 22 wrap around the cylinder 21. A grommet 23 encircles the spring 22 and the cylinder 21. The biasing means is mounted in an upper plate seat 24 and a lower plate seat 25. Similarly, the aft biasing means comprises rubber cylinder 26, spring 27 and grommet 28 resting in upper plate seat 29 and lower plate seat 30.

A bolt 40 can be used to secure the foot to the leg as an added safety feature in a manner to be described below. The bolt has associated therewith washer 41, rubber grommet 42, and washer 43. The bolt extends through slots 44 and 45 in the lower and upper plates, respectively, and is secured in a threaded unit such as nut 46 or the like, which is embedded in or otherwise secured to the leg portion 11 of the artificial leg 9.

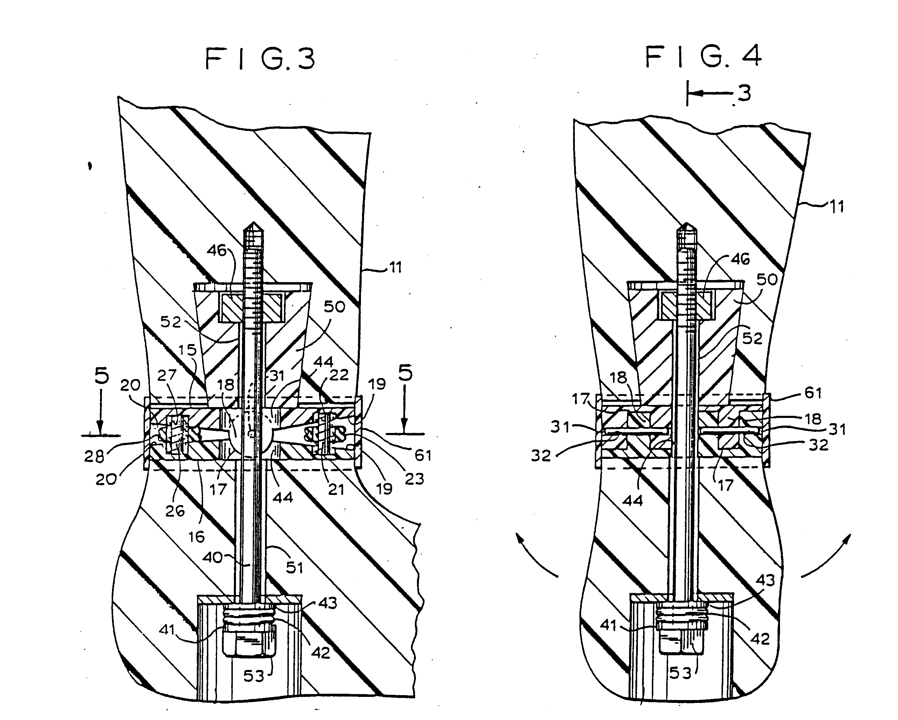

In one embodiment, the nut 46 is in the nature of a helicoil and is embedded in a plug insert 50 which is centrally located in the base of the leg 11 (Fig. 3). The bolt 40 passes through a bore 51 which extends through the heel of the foot 12 and is aligned with the slot 44 in the lower plate 14 (Fig. 5) and the slot 45 in the upper plate 13. The bolt 40 continues through the bore 52 in the plug 50 and threadably engages the nut 46 which is embedded in the plug 50. The head 53 of the bolt 40, with washer 41, grommet 42, and washer 43, rest in a seat 60 hollowed from the heel of the foot 12.

One or both of the bores 51 and 52 will generally have a diameter in excess of the diameter of the bolt such that the bolt will have a range of motion within the bore when the foot is rotated. This design is preferable when a bolt made from an especially rigid material is used. The bores may have a substantially oval cross-section, aligned with the slots in the plates, as shown by the different clearances evident in Figs. 3 and 4, which are oriented at right angles to one another about the longitudinal axis of the bolt.

During the walking motion, as the foot is lowered to the ground, the heel will commonly make contact first. This creates a force which acts to rotate the heel about the hinge axis 33 and to thereby move aft surfaces 20 towards one another. As this rotation takes place, cylinder 26 and spring 27 are compressed and thereby absorb some of the shock of impact. As the aft surfaces 20 move closer to one another, the cylinder 26 becomes larger in diameter as it is compressed. Its perimeter begins to press on the coils of the spring 27 such that compression of the spring coils is retarded and an increased biasing effect is created whereby further rotation about the hinge axis is more severely resisted. It can therefore be seen that when the leg is vertical and the foot is horizontal, there will be only minor resistance to bending, allowing a certain degree of freedom about the perpendicular. When bending is more severe, however, greater resistance is encountered, which helps to both align the foot relative to leg and also to absorb the shocks and strains which are associated with greater bending. As rotation of the plates continues, the aft surface 20 engage grommet 28 which gentlely stops further rotation without jarring.

The action of the biasing means in the fore half of the ankle is similar to that described above for the aft biasing means except that forward rotation should generally be more extensive than rearward rotation. The slope of the fore surfaces 19, and thus the included angle therebetween, is preferably greater than that of the aft surfaces 20 so that greater bending can occur in the forward direction than in the rearward direction. This action simulates that of the human ankle. Preferably, the sloped surfaces 19 coact with the fore biasing means to allow forward bending of the leg towards the foot of approximately 15°, while the aft sloped surfaces 20 and aft biasing means are so constructed as to allow approximately 6° rearward bending. Greater bending increases the risk of falls as the center of gravity moves with bending away from a steady position directly above the feet.

It would be apparent to one skilled in the art that alternative means to stop rotation of the plates at a predetermined position could be used in place of the sloped surfaces and the grommet. For example, abutting surfaces at the fore and aft most peripheries could be fashioned into the plates to perform this function. Similarly, the slope of the plates could be reduced or eliminated altogether in favor of a larger hinge, which would allow a similar degree of rotation.

During the bending action, the bolt 40 moves forwards or rearwards in the slots 44 and 45 and in one or both of the bores 51 and 52 such that the presence of the bolt does not interfere with the bending operation. Shear stresses brought on the bolt by the bending operation are absorbed partially by the grommet 42.

The hinge members 17 and 18, and pins 32 are built with sufficient tolerance to allow a small lateral rotation of the foot in either direction as indicated by the arrows in Fig. 4. This rotation is preferably approximately 2° in either direction from the vertical. As before, the bolt has sufficient lateral spacing in the slots 44 and 45 and in one or both of the bores 51 and 52 to allow this rotation, and any shear stress on the bolt is again partially taken up by the grommet 42.

The upper and lower plates are constructed preferably of nylon. Any durable plastic or other material which would be known to those skilled in the prosthetic art could be used. The cylinders 21 and 26, and the grommets 23, 28, and 42 can be made of any suitable elastomer which would be known to those skilled in the art. Preferable rubbers would be those known in the art as "80 gravity" rubbers. The springs 22 and 27, pins 32, bolt 40, washers 41 and 43, and nut 46, may be made of metals known to those skilled in the art, but preferably are fashioned from a quality stainless steel. The plug insert 50 is constructed as is known in the art but typically is made from willow wood.

Installation of the prosthetic ankle is simple and inexpensive. The prosthetic foot is sawed from the leg approximately where the human ankle would be located. Approximately one-half inch must be removed from the leg and approximately one-quarter inch must be removed from the foot to make room for the ankle. The plug 50 with the bore 52 and the nut 46 is inserted into a suitable space fashioned in the leg 11. Bolt seat 60 is fashioned in the heel of the foot 12 and the bore 51 is drilled through the foot. The lower plate 14 and upper plate 13 are installed at the top of the foot 16 and bottom of the leg 15 such that slots 44 and 45 align with the bolt bores 51 and 52.

The plates are secured to the leg and the foot through means known in the art. Preferably holes will be drilled as seats for screws (not shown in the drawings). The location of the holes is left to the discretion of the professional prosthetic installer, although a location between the biasing seats 24-25 and 29-30, and the slots 44 and 45 would do.

Once the plates are attached, the hinge members are interfitted with the biasing means in place and pins 32 are placed in the bores formed by cores 31. The pins 32 may include securing means known in the art such as split outer ends which wedge into the cores to secure the pins. The bolt 40 with washer 41, grommet 42, and washer 43 may then be slid through the bore 51, slots 44 and 45, into bore 52, and threadably engaged to nut 46 to thoroughly secure the foot 12 to the leg 11.

The presence of the bolt 40 is not necessary for operation of the invention. The pins 32 generally will be sufficient to hold the ankle together. It is an added safety feature which would hold the foot to the leg in the unlikely event that an element of the ankle device should fail.

A cosmetic covering 61 (Fig. 3) can be fitted around the prosthetic ankle to provide a more natural appearance. Such a covering would typically be made of pink vinyl and installed by methods known to those skilled in the art.

The present invention provides an inexpensive prosthetic ankle which can be easily and inexpensively installed in conventional rigid artificial legs. It is fashioned to allow bending similar to the range of motion allowed by the human ankle. It includes biasing means which absorb shock and act to return the foot to a natural position perpendicular to the leg. The biasing means are progressively resistant as more bending is encountered. Grommets stop bending, without jarring, at a point which simulates the range of motion of the human ankle but also helps to prevent loss of balance. The ankle is fashioned from durable materials which are nonetheless inexpensive to fashion and install. A bolt can be provided to insure that the foot remains attached to the leg in the unlikely event of a failure of an ankle element.

This invention may be embodied in other specific forms without departing from the spirit or essential attributes thereof, and accordingly, reference should be made to the appended claims, rather than to the foregoing specification, as indicating the scope of the invention.

## Claims

1. A prosthetic ankle device for movably attaching a lower leg part (11) to a foot part (12), the device to displace at least a part of a prosthetic leg, the device separating the lower leg part (11) and the foot part (12) from one another, the lower leg part (11) and foot part (12) defining facing surfaces (15, 16), the device comprising:

   upper and lower plates (13, 14) conforming in shape and adapted for attachment to said facing surfaces (15, 16) of said lower leg and foot parts (11, 12), respectively,

   said upper and lower plates (13, 14) together forming hinge means connecting said plates, said hinge means defining a lateral axis of rotation (33) for the foot portion and the lower leg portion (11) relative to one another, said hinge means being divided to form a recess in said upper and lower plates (13, 14)

   resilient biasing means (21, 22, 23; 26, 27, 28) disposed between said plates (13, 14) on both sides of said lateral axis (33) for progressively resisting rotation about said axis, whereby natural foot rotation about an ankle is simulated with respect to cushioning of foot strikes without risking loss of balance; and

   auxiliary bolt means (40) for securing said foot part (12) adjustably to said lower leg part (11) extending longitudinally through bores (51, 52) in the foot and leg parts (11, 12) and through said recess of the hinged plates (13, 14), which bores (51, 52) are aligned with said recess, and extending substantially through the lateral axis (33), while allowing said rotation.

2. The prosthetic ankle device of claim 1, wherein the hinge means connecting the upper and lower plates (13, 14) include spaced ridge shapes (18) on each of the plates disposed in corresponding spaced troughs (17) in the other of the plates, the ridge shapes (18) of the upper and lower plates being interleaved, bear against one another over a substantial area and having a lateral bore (31) along the said axis (33), and at least one hinge pin (32) extending through the ridge shapes (18).

3. The prosthetic ankle device of one of the preceding claims, further comprising means for limiting rotation in a backward direction more than in a forward direction, whereby the risk of toppling backwards is reduced without limiting forward rotation, whereby a full range of natural foot movement may be safely achieved.

4. The prosthetic ankle device according to one of claims 1-3, wherein said upper and lower plates (13, 14) have two pairs of opposed surfaces, one pair (19, 20) being on each side of said lateral axis (33), said pairs of surfaces defining oppositely directed included angles, said included angles defining a range of rotation about said lateral axis (33).

5. The prosthetic ankle device of claim 1, wherein said auxiliary bolt means (40) for securing said foot part (12) to said lower leg part (11) cooperate with the recess, in form of a slot (44) disposed fore and aft of the lateral axis (33) along and through each of said upper and lower plates (13, 14) and includes

   a first bore (51) extending through said foot part (12) to said slot (44), through said lower plate (14),

   and a second bore (52) extending through said upper plate (13) into said lower leg part (11);

   a unit (45, 50) adapted to receive the auxiliary bolt means (40), disposed at a closed end of said second bore (52);

   the auxiliary bolt means (40) passing into said heel and through said first bore (51), said slot (44) and said second bore (52), the auxiliary bolt means (40) having an end secured in place by said unit (46, 50) and an opposite end with a bolt head (53); and,

   resilient means disposed between the auxiliary bolt means (40) and at least one of the foot part (18) and the leg part (11), relative movement of said auxiliary bolt means (40) and said slots (44) during pivoting of the auxiliary bolt means (40) about the resilient means accommodating the full range of natural foot rotation, whereby the lower leg and foot portions may be securely connected without impairing operation of the device.

6. The prosthetic ankle device of one of the preceding claims, wherein for the adjustment of a lateral rotation said resilient biasing means includes a rubber grommet (42) and washers (41, 43) on each side of said grommet (42).

7. The prosthetic ankle device of claim 2, wherein said hinge means connecting the upper and lower plates (13, 14) are connected with sufficiently loose tolerances to one another and to said pin (32) to allow a small lateral rotation of said lower plate (14) up to about two degrees in either direction with respect to said upper plate (13).

8. The prosthetic ankle device of claim 1, wherein the ends of the auxiliary bolt means (40) are fixed at the leg (11) and at the foot (12), the auxiliary bolt means (40) being resilient.

9. The prosthetic ankle device of claim 1, wherein said biasing means comprises a cylinder (21, 26) constructed of an elastomeric material around which is wrapped coils of a spring (22, 27), an elastomeric grommet (21, 28) encircling said spring (22, 27) and said cylinder (21, 26), said cylinder (21, 26) being seated in bores (24, 25, 29, 30) in said upper plate (13) and said lower plate (14).

10. The prosthetic ankle device of claim 1, wherein the lower leg (11) is rotatable backwards relative to said foot (12) by up to approximatly six degrees, and rotatable forward relative the foot (12) by up to approximately fifteen degrees.

**Revendications**

1. Dispositif de prothèse de cheville pour relier de manière amovible une partie de jambe inférieure (11) à une partie de pied (12), le dispositif devant déplacer au moins une partie d'une prothèse de jambe, le dispositif séparant la partie de jambe inférieure (11) et la partie de pied (12) l'une de l'autre, la partie de jambe inférieure (11) et la partie de pied (12) délimitant des surfaces opposées (15, 16), le dispositif comprenant :
   - des plaques supérieure et inférieure (13, 14) ayant des formes correspondantes conçues pour être fixées sur lesdites surfaces opposées (15, 16) desdites parties de jambe inférieure et de pied (11, 12), respectivement.
   - lesdites plaques supérieure et inférieure (13, 14) formant ensemble des moyens d'articulation reliant lesdites plaques, lesdits moyens d'articulation définissant un axe latéral de rotation (33) pour la partie de pied et la partie de jambe inférieure (11) l'une par rapport à l'autre, lesdits moyens d'articulation étant divisés pour former un évidement (14) dans lesdites plaques supérieure et inférieure (13, 14),
   - des moyens élastiques de sollicitation (21, 22, 23; 26, 27, 28) placés entre lesdites plaques (13, 14) des deux côtés dudit axe latéral (33) pour s'opposer progressivement à une rotation autour dudit axe, de manière que la rotation naturelle du pied autour d'une cheville soit simulée du point de vue de l'amortissement des mouvements du pied sans risque de perte d'équilibre ; et
   - des moyens de boulons auxiliaires (40) pour fixer de manière réglable ladite partie de pied (12) à ladite partie de jambe inférieure (11) s'étendant longitudinalement dans des alésages (51, 52) des parties de pied et de jambe (11, 12) et traversant ledit évidement des plaques articulées (13, 14), lesquels alésages (51, 52) sont alignés avec ledit évidement, et traversent sensiblement l'axe latéral (33), permettant ainsi ladite rotation.

2. Dispositif de prothèse de cheville selon la revendication 1, dans lequel les moyens d'articulation reliant les plaques supérieure et inférieure (13, 14) comportent des formes de bosses espacées (18) sur chacune des plaques qui se placent dans des creux espacés de manière correspondante (17) de l'autre des plaques, les formes de bosses (18) des plaques supérieure et inférieure étant intercalées et appuyant l'une sur l'autre sur une surface notable et étant traversées par un alésage latéral (31) le long dudit axe (33), et au moins un goujon d'articulation (32) traversant lesdites formes de bosses (18).

3. Dispositif de prothèse de cheville selon l'une des revendications précédentes, comprenant en outre des moyens pour limiter la rotation dans la direction arrière davantage que dans la direction avant, de manière à réduire le risque de basculement vers l'arrière sans limiter la rotation en avant, afin d'assurer ainsi en sécurité une gamme complète de mouvements naturels du pied.

4. Dispositif de prothèse de cheville selon l'une des revendications 1 à 3, dans lequel lesdites plaques supérieure et inférieure (13, 14) comportent deux paires de surfaces opposées, l'une des paires (19, 20) se trouvant de chaque côté dudit axe latéral (33), lesdites paires de surface définissant des angles intérieurs directement opposés, lesdits angles intérieurs définissant une plage de rotation autour dudit axe latéral (33).

5. Dispositif de prothèse de cheville selon la revendication 1, dans lequel lesdits moyens de boulons auxiliaires (40) pour maintenir ladite partie de pied (12) à ladite partie de jambe inférieure (11) coopèrent avec l'évidement, sous forme d'une fente (44) pratiquée en avant et en arrière de l'axe latéral (33) dans et à travers chacune desdites plaques supérieure et inférieure (13, 14) et comporte

- un premier alésage (51) traversant ladite partie de pied (12) jusqu'à ladite fente (44), à travers ladite plaque inférieure (14),

et un second alésage (52) traversant ladite plaque supérieure (13) et pénétrant dans ladite partie de jambe inférieure (11) ;

une pièce (45, 50) adaptée pour recevoir les moyens de boutons auxiliaires (40), placée à l'extrémité fermée dudit second alésage (52) ;

les moyens de boulons auxiliaires (40) pénétrant dans ledit talon et traversant ledit premier alésage (51), ladite fente (44) et ledit second alésage (52), lesdits moyens de boulons auxiliaires (40) ayant une extrémité maintenue en place par ladite pièce (46, 50) et une extrémité opposée présentant une tête de boulon (53) ; et

des moyens élastiques placés entre les moyens de boulons auxiliaires (40) et au moins une parmi la partie de pied (18) et la partie de jambe (11), le mouvement relatif desdits moyens de boulons auxiliaires (40) et desdites fentes (44) au cours du pivotement desdits moyens de boulons auxiliaires (40) par rapport aux moyens élastiques permettant la gamme complète de rotation naturelle du pied, de manière que les parties de jambe inférieure et de pied puissent être reliées en sécurité sans gêner le fonctionnement du dispositif.

6. Dispositif de prothèse de cheville selon l'une quelconque des revendications précédentes, dans lequel lesdits moyens élastiques de sollicitation comportent à des fins de réglage de la rotation latérale un manchon en caoutchouc (42) et des rondelles (41, 43) de chaque côté dudit manchon (42).

7. Dispositif de prothèse de cheville selon la revendication 2, dans lequel lesdits moyens d'articulation reliant les plaques supérieure et inférieure (13, 14) sont assemblés entre eux et audit axe (32) avec des tolérances suffisamment lâches pour permettre une légère rotation latérale de ladite plaque inférieure (14) pouvant atteindre environ 2 degrés dans chacune des directions par rapport à ladite plaque supérieure (13).

8. Dispositif de prothèse de cheville selon la revendication 1, dans lequel les extrémités desdits moyens de boulons auxiliaires (40) sont fixées à la jambe (11) et au pied (12), lesdits moyens de boulons auxiliaires (40) étant élastiques.

9. Dispositif de prothèse de cheville selon la revendication 1, dans lequel ledit moyen de sollicitation comporte un cylindre (21, 26) fabriqué en un matériau élastomère autour duquel sont enroulées les spires d'un ressort (22, 27), un manchon en élastomère (21, 28) entourant ledit ressort (22, 27) ainsi que ledit cylindre (21, 26), ledit cylindre (21, 26) étant logé dans des alésages (24, 25, 29, 30) de ladite plaque supérieure (13) et de ladite plaque inférieure (14).

10. Dispositif de prothèse de cheville selon la revendication 1, dans lequel la jambe inférieure (11) peut être entraînée en rotation vers l'arrière par rapport audit pied (12) d'un angle maximal de six degrés, et en rotation en avant par rapport au pied (12) d'un maximum d'environ quinze degrés.

**Patentansprüche**

1. Fußgelenksprothese zum beweglichen Anbringen eines unteren Beinteiles (11) an einem Fußteil (12), wobei die Einrichtung zur Beweglichkeit zumindest eines Teils eines prothetischen Beines ausgebildet ist, die Einrichtung das untere Beinteil (11) und das Fußteil (12) voneinander trennt, das untere Beinteil (11) und Fußteil (12) einander zugekehrte Oberflächen (15, 16) aufweist, wobei die Einrichtung umfaßt:

obere und untere Platten (13, 14), die in ihrer Form übereinstimmen und zur jeweiligen Anbringung an den einander zugekehrten Oberflächen (15, 16) der unteren Bein- und Fußteile (11, 12) angepaßt sind,

die oberen und unteren Platten (13, 14), die zusammen Gelenkmittel bilden, die die Platten verbinden, wobei die Gelenkmittel eine drehbare Querachse (33) für den Fußabschnitt und den unteren Beinabschnitt (11) bezüglich einander bestimmen, wobei die Gelenkmittel geteilt sind, um eine Aussparung in den oberen und unteren Platten (13, 14) zu bilden,

nachgiebige Beaufschlagungsmittel (21, 22, 23; 26, 27, 28), die zwischen den Platten (13, 14) auf beiden Seiten der Querachse (33) angeordnet sind, um der Drehung um die Achse zunehmend zu widerstehen,

wobei die natürliche Fußdrehung um einen Fußknöchel simuliert ist, in bezug auf die Dämpfung von Fußstößen ohne zu riskieren, das Gleichgewicht zu verlieren; und

Hilfs-Bolzenmittel (40), die den Fußteil (12) einstellbar an dem unteren Beinteil (11) sichern, sich längs durch Bohrungen (51, 52) in den Fuß-und Beinteilen (11, 12) und durch die Aussparung der Gelenkplatten (13, 14) erstrecken, wobei die Bohrungen (51, 52) mit den Aussparungen fluchtend sind und sich im wesentlichen durch die Querachse (33) erstrecken, während die Drehung ermöglicht ist.

2. Fußgelenksprothese nach Anspruch 1, wobei die Gelenkmittel, die die oberen und unteren Platten (13, 14) verbinden, beabstandete Rippenformen (18) auf jeder der Platten enthalten, die in entsprechend beabstandeten Mulden (17) in den anderen Platten angeordnet sind, wobei die Rippenformen (18) der oberen und unteren Platten ineinander eingreifen, sich über einen wesentlichen Bereich gegenseitig tragen und eine Querbohrung (31) entlang der Achse (33) und zumindest einen Gelenkstift (32), der sich durch die Rippenformen (18) erstreckt, aufweisen.

3. Fußgelenksprothese nach einem der vorhergehenden Ansprüche, die weitere Mittel zur Drehungsbegrenzung, in Rückwärtsrichtung mehr als in Vorwärtsrichtung, umfaßt, wobei die Gefahr des Rückwärtskippens, ohne die Vorwärtsdrehung zu begrenzen, vermindert ist, wobei ein voller Bereich der natürlichen Fußbewegung sicher erzielt werden kann.

4. Fußgelenksprothese nach einem der Ansprüche 1 bis 3, wobei die oberen und unteren Platten (13, 14) zwei Paar sich gegenüberliegenden Oberflächen aufweisen, wobei ein Paar (19, 20) auf jeder Seite der Querachse (33) ist, wobei die Paare der Oberflächen entgegengesetzt gerichtete eingeschlossene Winkel aufweisen, wobei die eingeschlossenen Winkel den Bereich der Drehung um die Querachse (33) bestimmen.

5. Fußgelenksprothese nach Anspruch 1, wobei die Hilfs-Bolzenmittel (40), die den Fußteil (12) an das untere Beinteil (11) sichern, mit der Aussparung zusammenwirken, die in Form eines Schlitzes (44), der vor und hinter der Querachse (33), längs und durch jede der oberen und unteren Platten (13, 14) angeordnet ist und enthält

eine erste Bohrung (51), die sich durch das Fußteil (12) zu dem Schlitz (44) durch die untere Platte (14) erstreckt,

und eine zweite Bohrung (52), die sich durch die obere Platte (13) in das untere Beinteil (11) erstreckt;

eine Einheit (45, 50), zur Aufnahme der Hilfs-Bolzenmittel (40), die in einem geschlossenen Ende der zweiten Bohrung (52) angeordnet ist;

die Hilfs-Bolzenmittel (40), die in die Ferse hineinragen und durch die erste Bohrung (51), den Schlitz (44) und die zweite Bohrung (52) hindurch, wobei die Hilfs-Bolzenmittel (40) ein Ende, das durch die Einheit (46, 50) in seiner Position gesichert ist, und ein gegenüberliegendes Ende mit einem Bolzenkopf (53), aufweisen; und,

nachgiebige Mittel, die zwischen Hilfs-Bolzenmittel (40) und Fußteil (18) und/oder Beinteil (11) angeordnet sind, wobei eine Relativbewegung der Hilfs-Bolzenmittel (40) und der Schlitze (44), die während des drehbaren Lagerns der Hilfs-Bolzenmittel (40) um die nachgiebigen Mittel erfolgt, sich an den vollen Umfang der natürlichen Fußdrehung anpassen, wobei die unteren Bein- und Fußabschnitte sicher miteinander verbunden sind, ohne die Wirkung der Vorrichtung zu beinträchtigen.

6. Fußgelenksprothese nach einem der vorhergehenden Ansprüche, wobei zur Einstellbarkeit der seitlichen Drehung die nachgiebigen Beaufschlagungsmittel einen Gummiring (42) und Scheiben (41, 43) auf jeder Seite der Ringe (42) enthalten.

7. Fußgelenksprothese nach Anspruch 2, wobei die Gelenkmittel, die die oberen und unteren Platten (13, 14) verbinden, mit ausreichend großen Toleranzen zueinander und zu dem Stift (32) verbunden sind, um eine geringe seitliche Drehung der unteren Platte (14) um bis zu 2° in beide Richtungen bezüglich der oberen Platte (13) zu erlauben.

8. Fußgelenksprothese nach Anspruch 1, wobei die Enden der Hilfs-Bolzenmittel (40) an dem Bein (11) und dem Fuß (12) befestigt sind, wobei die Hilfs-Bolzenmittel (40) nachgiebig sind.

9. Fußgelenksprothese nach Anspruch 1, wobei die Beaufschlagungsmittel einen Zylinder (21, 26), der aus Elastomer-Material gebildet ist, um welchen Windungen einer Feder (22, 27) gewickelt sind, einen Elastomer-Ring (21, 28), der die Feder (22, 27) und den Zylinder (21, 26) umgibt, umfaßt, wobei der Zylinder (21, 26) in Bohrungen (24, 25, 29, 30) in der oberen Platte (13) und der unteren Platte (14) sitzt.

10. Fußgelenksprothese nach Anspruch 1, wobei das untere Bein (11) bezüglich des Fußes (12) um bis zu ungefähr 6° nach hinten und bezüglich des Fußes (12) um bis ungefähr 15° nach vorne drehbar ist.

FIG. 1

9

FIG. 5

11

F I G. 2

FIG. 4

FIG. 3